# EUROPEAN PATENT SPECIFICATION

(11) **EP 2 404 540 B1**
(45) Date of publication and mention of the grant of the patent: **04.01.2017**
(21) Application number: 09841137.4
(22) Date of filing: 18.09.2009
(51) Int. Cl.: A61B 1/04, A61B 1/00, A61B 1/12

(54) **MEDICAL DEVICE**
MEDIZINISCHE VORRICHTUNG
DISPOSITIF MÉDICAL

(30) Priority: 05.03.2009 JP 2009052367
(43) Date of publication of application: 11.01.2012
(73) Proprietor: Olympus Corporation, Tokyo 192-8507 (JP)
(72) Inventor: YAZAWA, Nobuyoshi, Hachioji-shi, Tokyo 192-8507 (JP); ASADA, Daisuke, Hachioji-shi, Tokyo 192-8507 (JP); NAKAJIMA, Sho, Hachioji-shi, Tokyo 192-8507 (JP); KARASAWA, Hitoshi, Hachioji-shi, Tokyo 192-8507 (JP)
(74) Representative: Schicker, Silvia
(86) International application number: PCT/JP2009/066405
(87) International publication number: WO 2010/100777

(56) References cited:
- EP-A1- 2 338 403
- JP-A- 60 203 229
- JP-A- H09 327 447
- JP-A- 2005 052 229
- JP-A- 2007 130 167
- JP-U- 52 063 985

## Description

### Technical Field

The present invention relates to a medical instrument provided with image pickup means for observing inside of a patient's body.

### Background Art

As is well known, endoscope apparatuses, which are medical instruments, are provided with an image pickup apparatus, which is image pickup means, designed to be introduced into a body cavity of a patient and perform various types of inspections and treatments of affected areas in the body based on observed images taken by the image pickup apparatus. Examples of such endoscopes include those introduced into digestive organs such as esophagus, stomach, large intestine and duodenum, which are tube cavities and tubes in the body, from the oral cavity or anus and those introduced into the abdominal cavity from the vicinity of the umbilical region by puncturing through the body wall.

Endoscope apparatuses for medical care are used in such an environment that dirt such as mucous membranes, filth or blood in the body is stuck to the observation window of the image pickup apparatus or vapor is stuck to the observation window of the image pickup apparatus in a humid environment in the body causing fogging of the observation window, and these deposits may thereby impair visibility, preventing clear images from being taken. Thus, conventionally, various proposals to remove deposits stuck to the observation window of image pickup apparatuses have been presented.

For example, Japanese Patent Application Laid-Open Publication No. 60-203229 discloses an endoscope incorporating a solid image pickup device which is provided with a member for wiping dirt or the like by contacting a transparent body such as a cover member that covers an observation window and one of the transparent body and the wiping member is movably arranged so as to be operable by the user so that fogging or deposits such as dirt stuck to the transparent body may be removed. Furthermore, for example, Japanese Patent Application Laid-Open Publication No. 2006-247261 discloses an endoscope including a rectification board provided on the surface of a cover member of an observation window facing an opening of a merging conduit that sends water, a cleaning liquid or the like, to improve cleaning and draining performance of the cover member.

Furthermore, for example, Japanese Patent Application Laid-Open Publication No. 2004-329292 discloses a small image pickup apparatus that sprays water stored in a water-retaining pack over a window in front of an image pickup lens from a nozzle to clean stuck gastric juices and residual contents in the stomach or the like.

For example, Japanese Patent Application Laid-Open Publication No. 2006-55275 also proposes an endoscope apparatus that selectively applies ultrasound vibration to a cover member, on an outer surface of which hydrophilic processed coating layer is formed, to prevent fogging of the outer surface of the cover member and remove dirt stuck, in addition to conventional cleaning means for removing dirt by spraying water and a cleaning liquid or the like over the observation window as described in Japanese Patent Application Laid-Open Publication No. 2006-247261 and Japanese Patent Application Laid-Open Publication No. 2004-329292 .

However, the endoscope described in Japanese Patent Application Laid-Open Publication No. 60-203229 has a problem that, when wiping and removing dirt on the surface of the transparent body by means of a wiping member, visibility in picking up images is obstructed and observation is interrupted. In addition, also in the case of the techniques described in Japanese Patent Application Laid-Open Publication No. 2006-247261 and Japanese Patent Application Laid-Open Publication No. 2004-329292 , water, cleaning liquid or the like sprayed on the surface of the cover member when removing dirt enters the field of view, thus interrupting observation.

Moreover, the technique of Japanese Patent Application Laid-Open Publication No. 2006-55275 has a problem that it takes time to remove deposits such as fogging and dirt stuck on the outer surface of the cover member.

EP 2 338 403 A1 discloses a medical instrument comprising a camera body that picks up an image of an object to be examined in a body. The medical instrument further comprises contamination removing means and a transparent cover member. The cover member is driven to make a sliding contact with the contamination removing means, and can thereby remove deposits stuck to an observation window formed in the camera body.

JPH09327447 A discloses a medical capsule comprising cylindrical inner and outer capsules. An image pickup means of the inner capsule is surrounded by an outer transparent capsule, which is rotated by moving means relative to the inner capsule.

The present invention has thus been made in view of the above-described problems and has an objective to provide a medical instrument capable of always obtaining a clear observation image without interfering with observation even when removing fogging and deposits such as dirt.

### Disclosure of Invention

### Means for Solving the Problem

To achieve the objective mentioned above, a medical instrument according to the present invention is defined in claim 1. It includes a body introduced into a body, image pickup means incorporated in the camera body for picking up an image of an object, a transparent cover member movably disposed at the camera body that always covers an image taking side of the image pickup means irrespective of a moving position, moving means that causes the cover member to move and cleaning means disposed outside an image pickup range of the image pickup means that performs cleaning by jetting a fluid over an opposed surface part of the cover glass moved to outside the image pickup range of the image pickup means.

### Brief Description of the Drawings

Fig. 1 is a perspective view illustrating a configuration of a camera set up in the abdominal cavity;
Fig. 2 is a partial cross-sectional view illustrating the camera set up in the abdominal cavity according to Fig. 1, which is set up in the abdominal cavity;
Fig. 3 is a cross-sectional view illustrating the configuration of the camera set up in the abdominal cavity according to Fig. 1;
Fig. 4 is a cross-sectional view illustrating the camera set up in the abdominal cavity according to Fig. 1, whose cover member has been slidingly moved;
Fig. 5 is a cross-sectional view illustrating the configuration of a further camera set up in the abdominal cavity provided with a wiper for wiping the surface of the sliding cover member;
Fig. 6 is a cross-sectional view illustrating a configuration of a further camera set up in the abdominal cavity;
Fig. 7 is a cross-sectional view along a line VII-VII in Fig. 6 illustrating the configuration of the camera set up in the abdominal cavity according to Fig. 6;
Fig. 8 is a cross-sectional view illustrating a configuration of a further camera set up in the abdominal cavity;
Fig. 9 is a cross-sectional view illustrating a configuration of a feeding drum of the camera set up in the abdominal cavity according to Fig. 6;
Fig. 10 is a cross-sectional view illustrating a configuration of a winding drum of the camera set up in the abdominal cavity according to Fig. 6;
Fig. 11 is a cross-sectional view illustrating a configuration of a further camera set up in the abdominal cavity;
Fig. 12 is a side view illustrating the configuration of the camera set up in the abdominal cavity according to Fig. 11;
Fig. 13 is a partial cross-sectional view illustrating a configuration of a cleaning nozzle of the camera set up in the abdominal cavity according to Fig. 11;
Fig. 14 is a cross-sectional view illustrating the configuration of the camera set up in the abdominal cavity with a wiping member provided at a cleaning nozzle;
Fig. 15 is a cross-sectional view illustrating a configuration of a further camera set up in the abdominal cavity;
Fig. 16 is a cross-sectional view illustrating the camera set up in the abdominal cavity according to Fig. 15 whose cleaning nozzle has been slidingly moved;
Fig. 17 is a partial cross-sectional view illustrating a configuration of a cleaning nozzle of the camera set up in the abdominal cavity according to Fig. 15;
Fig. 18 is a cross-sectional view illustrating the configuration of a further camera set up in the abdominal cavity;
Fig. 19 is a cross-sectional view illustrating a further camera set up in the abdominal cavity whose cleaning nozzle has been slidingly moved;
Fig. 20 is a cross-sectional view illustrating a configuration of a further camera set up in the abdominal cavity according to a sixth embodiment; and
Fig. 21 is a plan view of the camera set up in the abdominal cavity according to Fig. 20 viewed from the image taking direction.

### Best Mode for Carrying Out the Invention

Hereinafter, embodiments of the present disclosure will be described with reference to the accompanying drawings. In the following descriptions, a medical instrument provided with image pickup means used, for example, for a laparoscopic surgical operation will be taken as an example.

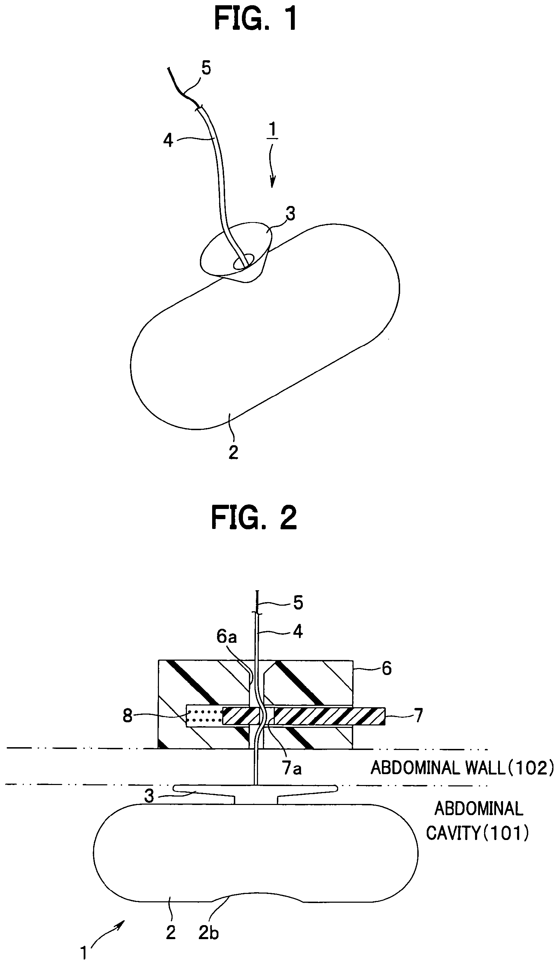

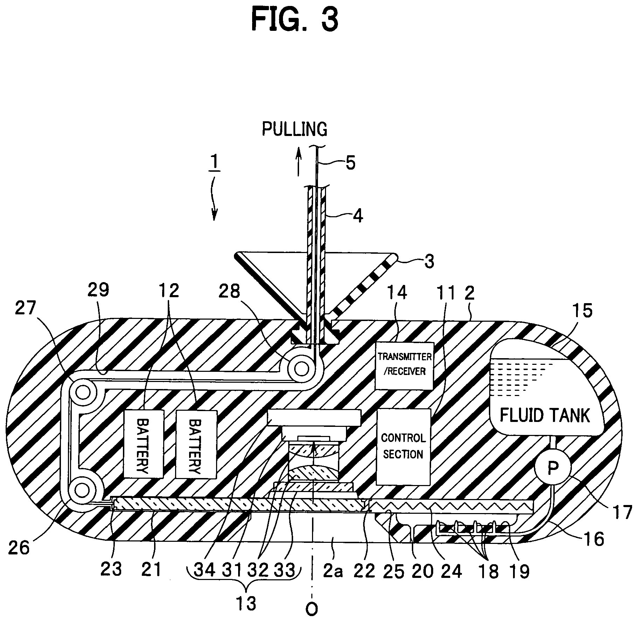

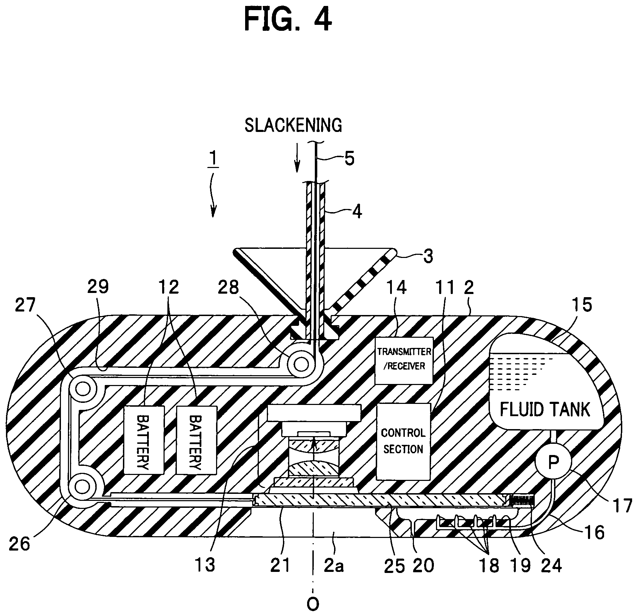

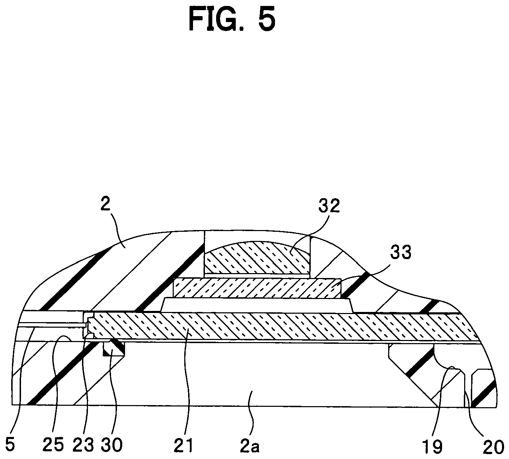

First, the camera set up in the abdominal cavity, which is a medical instrument of the present invention used for a laparoscopic surgical operation will be described based on Fig. 1 to Fig. 5 below. Fig. 1 is a perspective view illustrating a configuration of the camera set up in the abdominal cavity, Fig. 2 is a partial cross-sectional view illustrating the camera set up in the abdominal cavity, which is set up in the abdominal cavity, Fig. 3 is a cross-sectional view illustrating the configuration of the camera set up in the abdominal cavity, Fig. 4 is a cross-sectional view illustrating the camera set up in the abdominal cavity whose cover member has been slidingly moved and Fig. 5 is a cross-sectional view illustrating the configuration of the camera set up in the abdominal cavity provided with a wiper for wiping the surface of the sliding cover member.

As shown in Fig. 1 and Fig. 2, the camera set up in the abdominal cavity (hereinafter simply referred to as "camera") 1, which is a medical instrument, is mainly configured by including a camera body 2, which is a medical instrument body, having a capsule shape in which image pickup means (image pickup section) is incorporated, a suction cup 3 which is fixing means fitted into the camera body 2 and an abdominal wall fixing section, a flexible tube 4 which extends from the center of the suction cup 3 and an operation wire 5 which extends from an end of the flexible tube 4 and is inserted into the camera body 2 via the flexible tube 4. The camera body 2 is provided with an observation window 2a for observation, which is an opening formed in the underside of the perimeter opposite to the top side of the perimeter where the suction cup 3 is provided, in which the range of field of view of the camera 1 is included.

The camera 1 is used for a laparoscopic surgical operation and used to take images of a treatment region when treating organs or the like in an abdominal cavity 101, which is one of body cavities of the patient.

First, the camera 1 is introduced into the abdominal cavity 101 of the patient via a trocar (not shown) punctured through an abdominal wall 102. The flexible tube 4 is hooked onto the camera 1 by means of a puncture needle (not shown) or the like punctured into the abdominal cavity 101 and the flexible tube 4 is pulled out of the body so as to penetrate the abdominal wall 102.

Next, the flexible tube 4 of the camera 1 is passed through a hole 6a of a fixing unit 6 prepared on the abdominal side of the patient and pulled toward the abdominal wall 102 side. The camera 1 is lifted so that the camera 1 comes closer to the abdominal wall 102 and the flexible tube 4 is pulled toward the outside of the body until the suction cup 3 sticks fast to the inner surface of the abdominal wall 102. By the suction cup 3 sticking fast to the abdominal wall 102, the camera 1 is left indwelling and fixed in the abdominal cavity 101.

The fixing unit 6 is provided with a fixing lever 7 that fixes the flexible tube 4 of the camera 1 on the outside of the body. A hole 7a through which the flexible tube 4 passes is formed at some midpoint of the fixing lever 7 and the fixing lever 7 is urged in one sideward direction of the fixing unit 6 by a spring 8 provided in the fixing unit 6 so that the position of the hole 7a is deviated from the position of the hole 6a of the fixing unit 6.

That is, when the user pushes the fixing lever 7 into the fixing unit 6 up to a position at which the hole 6a of the fixing unit 6 substantially matches the hole 7a of the fixing lever 7 against the urging force of the spring 8, the user can easily pull the flexible tube 4. When the user stops pushing the fixing lever 7 into the fixing unit 6, the fixing lever 7 then slides by receiving the urging force of the spring 8.

Therefore, since the position of the hole 7a of the fixing lever 7 deviates from the position of the hole 6a of the fixing unit 6, the flexible tube 4 of the camera 1 that passes through the holes 6a and 7a is sandwiched and fixed in the fixing unit 6. Thus, the camera 1 is left indwelling and fixed in the abdominal cavity 101 in a stable state in which the suction cup 3, which is the abdominal wall fixing section, sticks fast to the abdominal wall 102.

Next, as described above, a more specific configuration of the camera 1 left indwelling and fixed in the body, the abdominal cavity 101 here, will be described in detail using Fig. 3 and Fig. 4.

The camera body 2 of the camera 1 incorporates a control section 11 which is control means constituting a controller, (here two) batteries 12 constituting a power supply section, an image pickup unit 13 which is image pickup means here and a transmitter/receiver 14 that transmits an image signal photoelectrically converted by the image pickup unit 13 to an external device.

Furthermore, the camera body 2 incorporates a fluid tank 15 storing water, a cleaning liquid or the like constituting cleaning means (a cleaning mechanical section), a fluid supply pipe 16 connected to the fluid tank 15 and a micro pump 17 inserted in the fluid supply pipe 16. One end of the fluid supply pipe 16 is connected to the fluid tank 15 and the other end is branched into a plurality of (here four) portions and a cleaning nozzle 18, which is a cleaning section, is disposed at each of the four branch ends. The cleaning nozzles 18 also constitute part of the cleaning means (cleaning mechanical section).

The four cleaning nozzles 18 are fixed to a bottom surface section of a cleaning tank 19, which is a space formed in the camera body 2, and jet water, a cleaning liquid or the like in the fluid tank 15 upward. That is, water, a cleaning liquid or the like stored in the fluid tank 15 is supplied to the fluid supply pipe 16 driven by the micro pump 17 and jetted from the four cleaning nozzles 18.

Furthermore, the cleaning tank 19 communicates with a drainage channel 20 which is a hole formed from the underside of the camera body 2 to discharge water, a cleaning liquid or the like jetted from the cleaning nozzles 18 to the outside of the camera body 2. This cleaning tank 19 is disposed to the side of the observation window 2a and on the right side when viewed from the front of the sheet in Fig. 3. That is, the four cleaning nozzles 18 disposed in the cleaning tank 19 are arranged at positions outside the field of vision of the image pickup unit 13.

Furthermore, a cover member 21, which is a plate like transparent member, is provided at the camera body 2 below the image pickup unit 13 so as to block the upper part of the observation window 2a, which is an opening, and cover the image taking side of the image pickup unit 13 in a manner freely movable sliding leftward/rightward. This cover member 21 has a predetermined length, and metallic fixing frames 22 and 23 are fixed at both ends thereof. Surface coating such as hydrophilic coating may be applied to the surface of the cover member 21.

One end of a coil spring 24, which is an elastic member, is fixed on one side; to the fixing frame 22 here, on the right side as viewed from the front of the sheet. One end of the operation wire 5 is fixed on the other side; to the fixing frame 23 here, on the left side as viewed from the front of the sheet.

A guide groove 25 is formed in the camera body 2 so that the cover member 21 may slide rectilinearly leftward and rightward. The above described coil spring 24 is accommodated in this guide groove 25 on the right side as viewed from the front of the sheet here.

That is, as shown in Fig. 3 and Fig. 4, the coil spring 24 is accommodated in the guide groove 25 located in the upper part of the cleaning tank 19. The coil spring 24 is set so as to urge the cover member 21 in a direction of pulling the cover member 21 rightward as viewed from the front of the sheet here. A predetermined length of the cover member 21 in the sliding direction is set so as to always block the observation window 2a and cover the image pickup unit 13 within the leftward/rightward sliding range.

Furthermore, the insertion direction of the operation wire 5 connected to the fixing frame 23 of the cover member 21 is changed into a crank shape here by three pulleys 26, 27 and 28 in the camera body 2 and the operation wire 5 is extended outward via the flexible tube 4. A passage 29 for the operation wire 5 to pass is formed in the camera body 2 and the pulleys 26, 27 and 28 are arranged in the respective corners of the passage 29.

The image pickup unit 13, which is image pickup means (image pickup section), is configured by including a solid image pickup device 31 such as CCD, CMOS, an image pickup substrate 32 on which the solid image pickup device 31 is mounted, a plurality of objective lenses 33 and a cover lens 34. The image pickup unit 13 condenses light of an object image shown by an image taking optical axis O to a light-receiving section of the solid image pickup device 31, photoelectrically converts the received object image and outputs an image signal to the control section 11. Furthermore, the camera body 2 is provided with an illumination unit (not shown) that irradiates the object with illumination light.

The control section 11 transmits an image signal from the transmitter/receiver 14 to a camera control unit (CCU) (not shown), which is an external device. The image taken by the image pickup unit 13 is subjected to image processing by the CCU and displayed on an outside monitor (not shown).

Furthermore, the control section 11 drives/controls the micro pump 17 according to a drive instruction signal from outside received by the transmitter/receiver 14. That is, the micro pump 17 is driven/stopped by the user operating an outside controller (not shown).

Furthermore, two batteries 12 constitute a power supply section to drive the control section 11, the image pickup unit 13, the transmitter/receiver 14, the micro pump 17 and the illumination unit (not shown). The two batteries 12 are electrically connected to the control section 11 via an electric cable (not shown) for power supply. The control section 11 is connected to the image pickup unit 13, the transmitter/receiver 14, the micro pump 17 or the like individually via communication cables (not shown) for power supply and exchange of signals.

The camera 1 configured as shown above is introduced into the abdominal cavity 101 as described above for a laparoscopic surgical operation and used by being left indwelling and fixed with the suction cup 3 sticking fast to the abdominal wall 102. When used, the camera 1 is normally used with the cover member 21 moved to the left along the guide groove 25 as viewed from the front of the sheet as shown in Fig. 3 here.

That is, the camera 1 is operated and held in a condition in which the operation wire 5 is pulled by the user and the cover member 21 is moved leftward as viewed from the front of the sheet here along the guide groove 25 against the urging force of the coil spring 24. The moving position and condition of the cover member 21 are held in this way and the diseased part in the abdominal cavity 101 is observed during normal operation.

During surgical treatment by laparoscopy, when fogging occurs on the surface of the cover member 21 exposed at the observation window 2a or dirt such as mucous membrane, blood is stuck and visibility is thereby impaired, the user slackens the operation wire 5 and the cover member 21 moves rightward as viewed from the front of the sheet here along the guide groove 25 by receiving the urging force of the coil spring 24 as shown in Fig. 4. That is, the cover member 21 which has moved by the urging force of the coil spring 24 moves above the cleaning tank 19. The operation wire 5 and the coil spring 24 for causing the cover member 21 to slide constitute moving means (moving section).

In this case, the user operates the controller (not shown) at hand to drive the micro pump 17 and causes water, a cleaning liquid or the like to jet from the four cleaning nozzles 18 over the surface of the cover member 21 on which fogging occurs or to which dirt such as mucous membrane, blood is stuck, that is, the surface opposed to the four cleaning nozzles 18. Thus, the surface of the cover member 21 which has moved above the cleaning tank 19 is cleaned with water, a cleaning liquid or the like, making it possible to thereby perform defogging or remove sticking mucous membrane, blood or the like.

When the cover member 21 is slidingly moved by the urging force of the coil spring 24 and the portion of the surface to which fogging, dirt or the like is stuck has been moved to the cleaning tank 19 side, the portion accommodated in the guide groove 25 opposite to the cleaning tank 19 comes to block the observation window 2a. That is, no fogging, dirt or the like is stuck to the exposed surface of the cover member 21 that covers the image taking side of the image pickup unit 13 so as to block the observation window 2a, allowing the image pickup unit 13 to take clear images.

After performing defogging or removing dirt such as mucous membrane, blood, the user operates the controller (not shown) at hand to stop the micro pump 17, pulls the operation wire 5 again so as to restore the state in which the cover member 21 has moved leftward along the guide groove 25 as shown in Fig. 3. The portion of the surface of the cover member 21 from which fogging or deposits such as dirt have been removed moves to the position where the portion of the surface is exposed so as to block the observation window 2a including the field of view of the image pickup unit 13, thus allowing the image pickup unit 13 to take clear images.

As described above, the camera 1 includes the slidable transparent cover member 21 which is exposed so as to always block the observation window 2a of the image pickup unit 13 that takes an image of the object to be examined and has a configuration in which when fogging, dirt or the like is stuck to the surface of the cover member 21, the user causes the cover member 21 to slidingly move, and can thereby clean fogging or deposits such as dirt from the surface outside the field of view of the image pickup unit 13. Furthermore, the camera 1 also has a configuration in which while cleaning fogging, dirt or the like stuck to part of the surface of the cover member 21, the other part of the surface of the cover member 21 to which no fogging, dirt or the like is stuck blocks the observation window 2a and moves into the field of view of the image pickup unit 13.

Thus, when visibility of the camera 1 is impaired, the user pulls the operation wire 5, cleans part of the surface of the cover member 21 to which dirt or the like is stuck and which has slidingly moved to the cleaning tank 19, then slackens the operation wire 5 and returns the operation wire 5 to its original position, and can thereby take a clear object image.

That is, the camera 1 has a configuration in which water, a cleaning liquid or the like for cleaning part of the surface of the cover member 21 never enters the observation area of the image pickup unit 13, and it is thereby possible to remove fogging or deposits such as dirt without interfering with observation and always obtain a clear observation image.

As shown in Fig. 5, the camera 1 may also be provided with a wiping member 30 such as a wiper that contacts the other part of the surface of the cover member 21 exposed so as to block the observation window 2a to wipe fogging, dirt or the like in the observation window 2a. In this way, while cleaning fogging, dirt or the like stuck to part of the surface of the exposed cover member 21 so as to block the observation window 2a in normal operation, even if fogging, dirt or the like is stuck to the other part of the surface of the cover member 21 which has been slidingly moved and exposed so as to block the observation window 2a, it is possible to wipe/remove such fogging, dirt or the like stuck to the other part of the cover member 21 with the wiping member 30 when the cover member 21 slidingly moves again for normal operation.

Furthermore, the cover member 21 is not limited to the configuration of slidingly moved by pulling/slackening of the operation wire 5, but, for example, it is also possible to adopt a configuration in which the cover member 21 is made to slidingly move electrodynamically using moving means (drive source) using a motor.

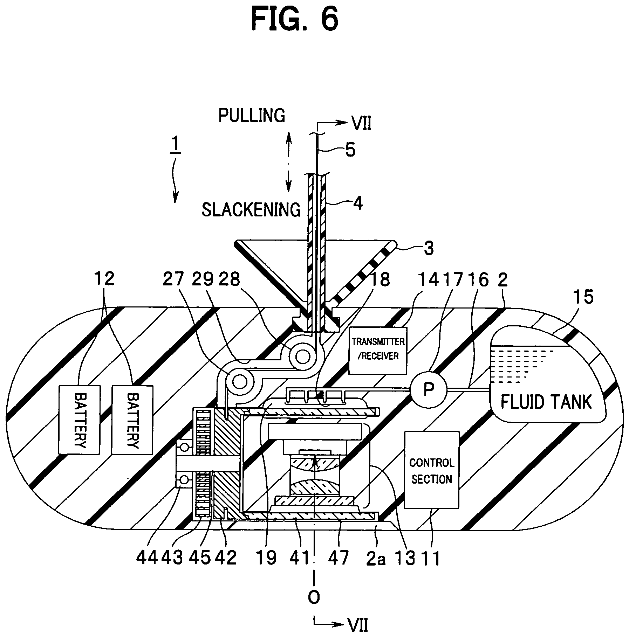

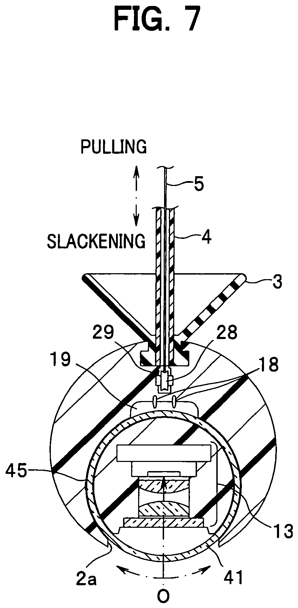

Next, a further camera set up in the abdominal cavity, which is a medical instrument according to the present invention, will be described based on Fig. 6 and Fig. 7. Fig. 6 is a cross-sectional view illustrating a configuration of the camera set up in the abdominal cavity and Fig. 7 is a cross-sectional view along a line VII-VII in Fig. 6 illustrating the configuration of the camera set up in the abdominal cavity. In the following descriptions, the same components as those in the camera set up in the abdominal cavity of the first embodiment will be assigned the same reference numerals and descriptions of those components and operations/effects will be omitted.

In the camera set up in the abdominal cavity (hereinafter also simply referred to as "camera") 1, a cover member 41 which is made to rotate by a pulling/slackening operation of the operation wire 5 is disposed so as to cover the periphery of the image pickup unit 13 as shown in Fig. 6 and Fig. 7.

To be more specific, the camera body 2 is provided with the cylindrical transparent cover member 41 which rotates around an axis parallel to the longitudinal axis. Surface coating such as hydrophilic coating may be applied to the surface of the cover member 41, too.

The cover member 41 is fitted into a rotation pulley 42, one end of which is rotatably disposed. The rotation pulley 42 is connected to an axial body 45 rotatably held by a bearing 44 which is fixed in the camera body 2. Furthermore, the operation wire 5 is wound around the rotation pulley 42. That is, when the user pulls the operation wire 5, the rotation pulley 42 rotates in a predetermined direction.

The operation wire 5 is inserted into the camera body 2 from the flexible tube 4, passed through the passage 29 as in the arrangement of Figs. 1 to 5 and the insertion direction thereof is changed by two pulleys 27 and 28.

A spiral spring 43 is connected to the axial body 45. The spiral spring 43 is disposed so as to generate a counter force in the axial body 45 in the rotation direction around the axial body 45 around which the rotation pulley 42 rotates by pulling of the operation wire 5 and produce energy for rotating in the reverse direction.

The operation wire 5, the rotation pulley 42 and the spiral spring 43 constitute moving means (moving mechanical section) that rotates the cover member 41.

The aforementioned cover member 41, the rotation pulley 42 fitted into the cover member 41 and supported on the axial body 45 and the spiral spring 43 connected to the axial body 45 are disposed in a cavity 47 having a channel-shaped cross section formed in the camera body 2 and cylindrically hollowed out in accordance with the rotation of the cover member 41. The cavity 47 is formed so as to surround the image pickup unit 13 in communication with the observation window 2a so that the rotating cover member 41 is always exposed while blocking the observation window 2a.

That is, the cover member 41 is disposed so as to surround the image pickup unit 13 and arranged in the camera body 2 so as to be exposed while blocking the observation window 2a at any rotation position.

Furthermore, a cleaning tank 19 is formed in the upper part of the cylindrical part of the cavity 47 in which the cover member 41 is disposed, a spout of which is directed downward so that the cleaning nozzle 18 can jet water, a cleaning liquid or the like toward the surface of the cover member 41. There are lateral two lines of cleaning nozzles 18 as shown in Fig. 7 and longitudinal four cleaning nozzles 18 along the longitudinal axis of the fluid supply pipe 16 as shown in Fig. 6, a total of eight cleaning nozzles 18 are provided in the cleaning tank 19.

Since the cleaning tank 19 provided with the cleaning nozzles 18 is also provided outside the field of view of the image pickup unit 13, the camera 1 can have a configuration in which water, a cleaning liquid or the like for cleaning the surface of the dirty cover member 41 never enters the observation area of the image pickup unit 13, it is possible to remove fogging or deposits such as dirt without interfering with observation and always obtain a clear observation image.

Furthermore, the rest of the configuration of the camera 1 is the same as that of the camera 1 of Figs. 1 to 5.

The camera 1 of the present embodiment configured as shown above is used by being introduced into the abdominal cavity 101 for a laparoscopic surgical operation and left indwelling and fixed with the suction cup 3 sticking fast to the abdominal wall 102 as in the case of the arrangement according Figs. 1 to 5.

During surgical treatment by laparoscopy, when visibility is impaired due to generation of fogging on or sticking of dirt such as mucous membrane, blood to the surface of the cover member 41 exposed while covering the image taking side of the image pickup unit 13 so as to block the observation window 2a, the user pulls the operation wire 5 to cause the cover member 41 to rotate around the axial body 45 in a predetermined direction. In this case, the surface of the portion of the cover member 41 hidden in the cavity 47, which is free of fogging, dirt or the like, moves to a position where the surface is exposed while blocking the observation window 2a. Thus, the camera 1 can take a clearer object image using the image pickup unit 13.

In this case, the user operates a controller (not shown) at hand to drive the micro pump 17 and cause the (here eight) cleaning nozzles 18 to jet water, a cleaning liquid or the like toward the surface of the cover member 41 on which fogging is generated or to which dirt such as mucous membrane, blood is stuck, that is, the surface opposed to the eight cleaning nozzles 18 as in the case of the arrangement according to Figs. 1 to 5. Thus, the surface of the cover member 41 which has rotatingly moved onto the cleaning tank 19 is cleaned with water, a cleaning liquid or the like, and it is possible to remove fogging or dirt such as mucous membrane, blood stuck thereto. The user can then stop the micro pump 17 using the controller (not shown) at hand.

Furthermore, when the user pulls the operation wire 5 and the surface of the cover member 41 exposed from the observation window 2a becomes dirty, the user slackens the operation wire 5. The axial body 45 then rotates by receiving the urging force of the spiral spring 43 and the surface part of the cover member 41 which has been washed free of dirt returns to the original position, that is, the position where the surface part of the cover member 41 is exposed from the observation window 2a. The user then performs the aforementioned operation on the dirty surface of the cover member 41 that rotates in the cleaning tank 19 for cleaning.

As described above, the camera 1 can easily rotate the surface of the cover member 41 exposed from the observation window 2a by pulling/slackening the operation wire 5 and always take a clear object image by repeating the cleaning of the surface of the cover member 41 located in the cleaning tank 19.

That is, the camera 1 has the same effect as that of the arrangement according to Figs. 1 to 5 and has a configuration in which water, a cleaning liquid or the like for cleaning part of the surface of the cover member 41 never enters the observation area of the image pickup unit 13, and thereby never interferes with observation and it is possible to remove fogging or deposits such as dirt and always obtain a clear observation image.

The cover member 41 is not limited to the configuration in which the cover member 41 is rotated by pulling/slackening the operation wire 5, but a configuration may also be adopted in which the cover member 41 is rotated using movable means (drive source) using, for example, a motor.

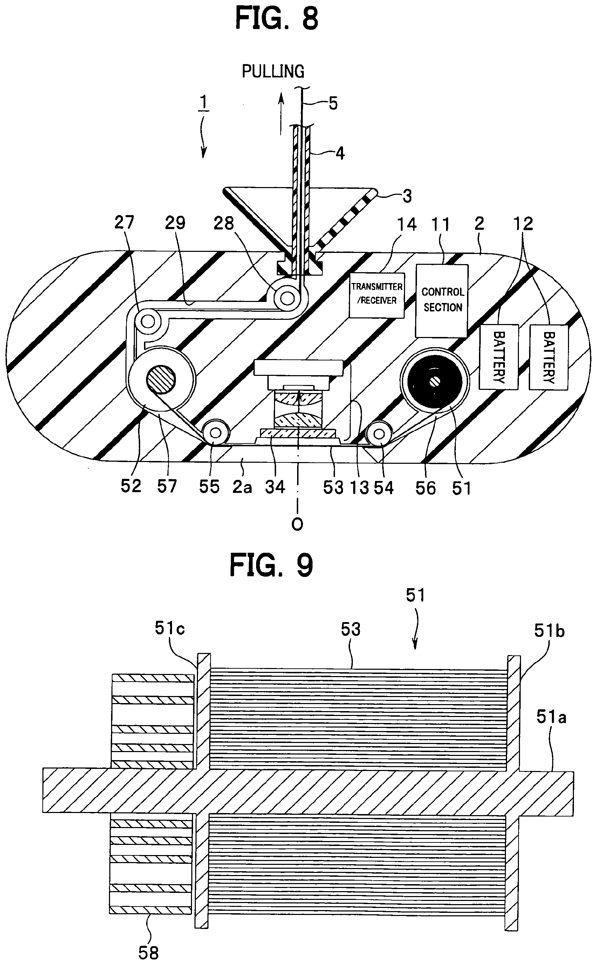

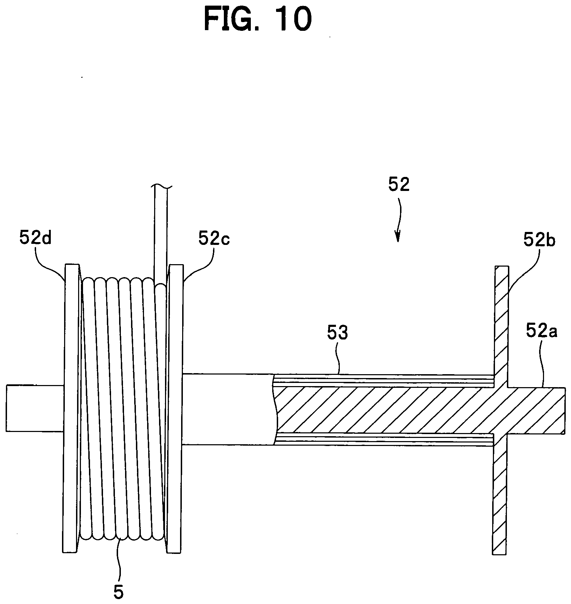

Next, a further camera set up in the abdominal cavity will be described based on Fig. 8 to Fig. 10. Fig. 8 is a cross-sectional view illustrating a configuration of the camera set up in the abdominal cavity, Fig. 9 is a cross-sectional view illustrating a configuration of a feeding drum and Fig. 10 is a cross-sectional view illustrating a configuration of a winding drum. In the following descriptions, the same components as those in the camera set up in the abdominal cavity of Figs. 1 to 5 will be assigned the same reference numerals and descriptions of those components and operations/effects will be omitted.

As shown in Fig. 8, the camera set up in the abdominal cavity (hereinafter also simply referred to as "camera") 1 has a configuration in which a transparent film 53 wound around a feeding drum 51 is wound up by a winding drum 52 by pulling the operation wire 5 and the transparent film 53 is thereby sent so as to pass on the surface side of a cover lens 34 of the image pickup unit 13.

To be more specific, drum accommodating sections 56 and 57 which are spaces for rotatably accommodating the feeding drum 51 and the winding drum 52 are formed in the camera body 2 of the camera 1, left and right so as to sandwich the image pickup unit 13. The drum accommodating sections 56 and 57 are formed so as to communicate with the observation window 2a. Rollers 54 and 55 are disposed on both sides of and in the vicinity of the observation window 2a with which the drum accommodating sections 56 and 57 communicate so that the transparent film 53 may rectilinearly pass along the surface of the cover lens 34 of the image pickup unit 13 .

The transparent film 53 flattened by the two rollers 54 and 55 is placed so as to block the observation window 2a and cover the image taking side of the image pickup unit 13. Surface coating such as hydrophilic coating may be applied to the surface of the transparent film 53.

As shown in Fig. 9, the feeding drum 51 is provided with two outward flanges 51b and 51c on a drum shaft 51a, and the transparent film 53 is wound between the two outward flanges 51b and 51c. The drum shaft 51a is pivotally supported so that the feeding drum 51 is freely rotatable in the drum accommodating section 56 formed in the camera body 2.

Furthermore, a spiral spring 58 connected to the drum shaft 51a is provided outside the one outward flange 51c. The spiral spring 58 is set so as to generate an urging force in a reverse direction against the rotation direction in which the feeding drum 51 sends out the transparent film 53. That is, the spiral spring 58 gives tension such that when the transparent film 53 wound up by the winding drum 52 blocks the observation window 2a and covers the image taking side of the image pickup unit 13, the transparent film 53 is always kept flat.

As shown in Fig. 10, regarding the winding drum 52, three outward flanges 52b, 52c and 52d are provided on a drum shaft 52a and the transparent film 53 is wound up between the two outward flanges 52b and 52c. Furthermore, the operation wire 5 is wound between the outward flanges 52b and 52c. That is, when the operation wire 5 is pulled, the winding drum 52 rotates so as to wind up the transparent film 53 in a predetermined direction.

Here, the operation wire 5 is also inserted into the camera body 2 from the flexible tube 4, passed through the passage 29, and the insertion direction thereof is changed by the two pulleys 27 and 28 as in the case of the second embodiment.

The rest of the configuration of the camera 1 is also the same as that of the camera 1 of Figs. 1 to 5.

In the camera 1 configured as shown above, when fogging, deposits such as dirt are stuck to the transparent film 53 which is exposed at the observation window 2a and covers the image taking side of the image pickup unit 13 and visibility is thereby impaired, the user pulls the operation wire 5 and thereby sends out the transparent film 53 to the observation window 2a. In other words, in the camera 1, when the user pulls the operation wire 5, the winding drum 52 rotates, winds up the transparent film 53 to which fogging, dirt or the like is stuck, a new part of the film wound around the feeding drum 51 free of fogging, dirt or the like is sent out so as to be exposed from the observation window 2a and cover the image taking side of the image pickup unit 13.

Thus, the camera 1 of the present embodiment has a configuration in which when fogging, dirt or the like is stuck to the transparent film 53 and visibility of the image pickup unit 13 is impaired, a clean part of the transparent film 53 can be sent out so as to be exposed at the observation window 2a and cover the image taking side of the image pickup unit 13 by pulling the operation wire 5, and it is thereby possible to always obtain a clear observation image without interfering with observation.

A configuration may also be adopted in which the feeding drum 51 wound with the transparent film 53 and the winding drum 52 for winding up the transparent film 53 are integrally formed into a cartridge type so as to be replaceable by being attached/detached to/from the camera body 2. Furthermore, the winding drum 52 is not limited to the configuration in which the winding drum 52 is rotated by pulling the operation wire 5 but may also be configured so as to be rotated electrodynamically driven by a motor.

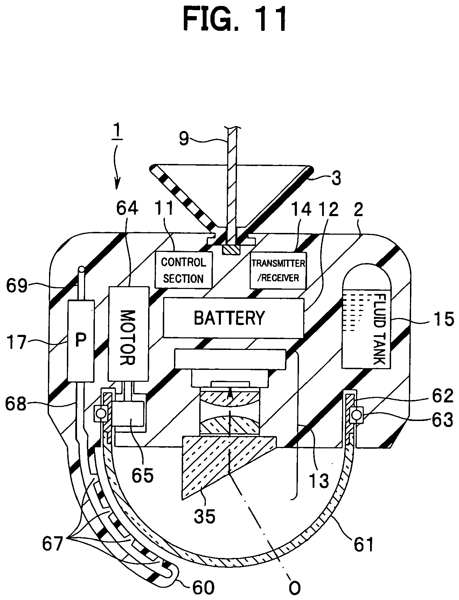

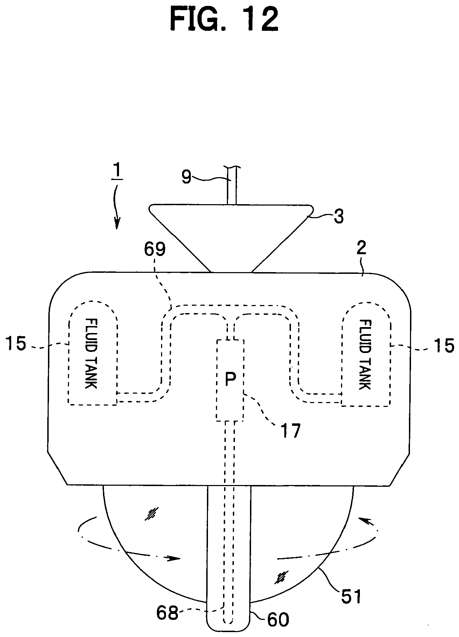

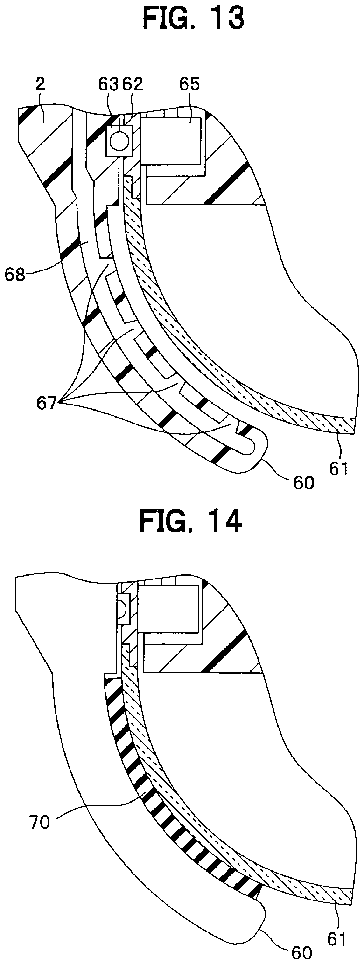

Next, a further camera set up in the abdominal cavity will be described based on Fig. 11 to Fig. 14 below. Fig. 11 is a cross-sectional view illustrating a configuration of the camera set up in the abdominal cavity, Fig. 12 is a side view illustrating a configuration of the camera set up in the abdominal cavity, Fig. 13 is a partial cross-sectional view illustrating a configuration of a cleaning nozzle and Fig. 14 is a cross-sectional view illustrating a configuration with a wiping member provided at a cleaning nozzle. In the following descriptions, the same components as those in the camera set up in the abdominal cavity of Figs. 1 to 5 will be assigned the same reference numerals and descriptions of those components and operations/effects will be omitted.

As shown in Fig. 11 to Fig. 13, the camera set up in the abdominal cavity (hereinafter also simply referred to as "camera") 1 has a configuration in which a transparent dome-shaped cover member 61 covering an image taking side of the image pickup unit 13 is rotatably disposed at the camera body 2.

To be more specific, the camera 1 is provided with no flexible tube and a wire 9 for lifting the camera body 2 extends from the suction cup 3 which is an abdominal wall fixing section.

Furthermore, the camera body 2 is provided with a cleaning nozzle 60 which extends in an arc shape along the outer surface of the cover member 61. The cleaning nozzle 60 constitutes one of cleaning means (cleaning mechanical section) and a fluid supply channel 68 for jetting a fluid is formed inside (see Fig. 13). Furthermore, the cleaning nozzle 60 is provided with a plurality of (here four) openings 67 for jetting a fluid that communicate with the fluid supply channel 68 arranged in parallel on a part facing the surface of the cover member 61 along the extending direction thereof.

The fluid supply channel 68 communicates with the micro pump 17 disposed in the camera body 2 and the micro pump 17 sends a fluid such as water, a cleaning liquid stored in the fluid tank 15. Furthermore, the micro pump 17 is connected to the fluid tank 15 via the fluid supply pipe 16.

There are two fluid tanks 15 in the camera body 2 as shown in Fig. 12. The two fluid tanks 15 are connected to branch ends of a branched fluid supply pipe 69 respectively.

The peripheral edge of the dome-shaped transparent cover member 61 is fitted and fixed to a base 62 rotatably disposed in the camera body 2 via a bearing 63. That is, the cover member 61 is rotatably disposed with respect to the camera body 2.

Furthermore, a roller 65 formed of an elastic member such as rubber of a motor 64 incorporated in the camera body 2 is held in pressure contact with the inner surface of the base 62 and the roller 65 rotates with respect to the camera body 2 together with the cover member 61 as the motor 64 rotates. A gear may be used instead of the roller 65 and in that case, a gear groove may be formed in the inner surface of the base 62.

Furthermore, the motor 64 constitutes moving means (drive source) and is driven/controlled by the control section 11. The motor 64 is driven/stopped under the control of the control section 11 by the user operating an outside controller (not shown) and thereby inputting a wireless instruction signal to the transmitter/receiver 14 as in the case of the drive/control described about the micro pump 17 of the arrangement according to Figs. 1 to 5. Thus, the cover member 61 is configured to rotate/stop through drive/control of the motor 64 with respect to the camera body 2.

The image pickup unit 13 of the present embodiment is provided with a prism 35 that causes the image taking optical axis O to refract instead of the cover lens so that the cleaning nozzle 60 deviates from the range of field of view. That is, the image pickup unit 13 has a so-called oblique-viewing configuration in which the image taking direction is inclined by a predetermined angle using the prism 35 so that the cleaning nozzle 60 does not enter the range of the field of view.

Furthermore, the rest of the configuration of the camera 1 is also the same as that of the camera 1 of the arrangement according to Figs. 1 to 5.

According to the camera 1 configured as above, when fogging, dirt or the like is stuck to the surface of the cover member 61 that covers the image taking side of the image pickup unit 13 and visibility is impaired, the motor 64 is driven to rotate the cover member 61 and the micro pump 17 is driven to jet water, a cleaning liquid or the like from the four openings 67 of the cleaning nozzle 60, and it is thereby possible to clean the surface of the cover member 61 that rotates. That is, when fogging, dirt or the like is stuck to the cover member 61 and visibility of the image pickup unit 13 is impaired, water, a cleaning liquid or the like is jetted from the cleaning nozzle 60 disposed outside the field of view of the image pickup unit 13, and the camera 1 can thereby be configured to be able to clean the surface of the cover member 61 without interfering with observation and always obtain a clear observation image.

The cleaning nozzle 60 may also be disposed so that a wiping member such as a wiper made of an elastic member such as rubber contacts the surface of the cover member 61 as shown in Fig. 14.

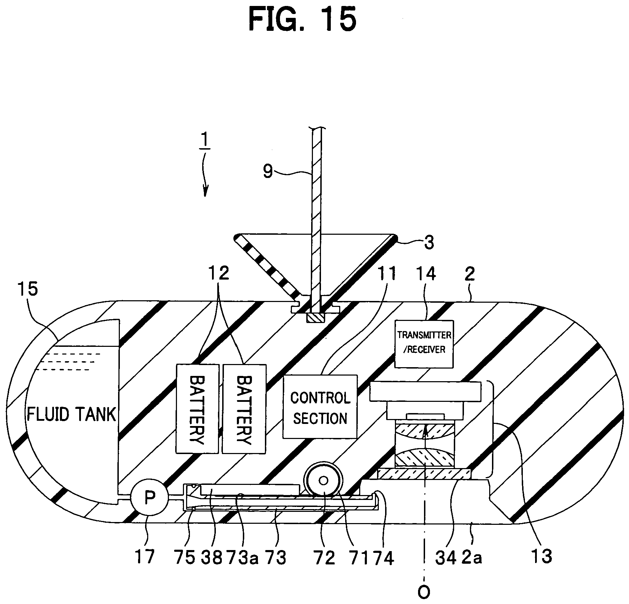

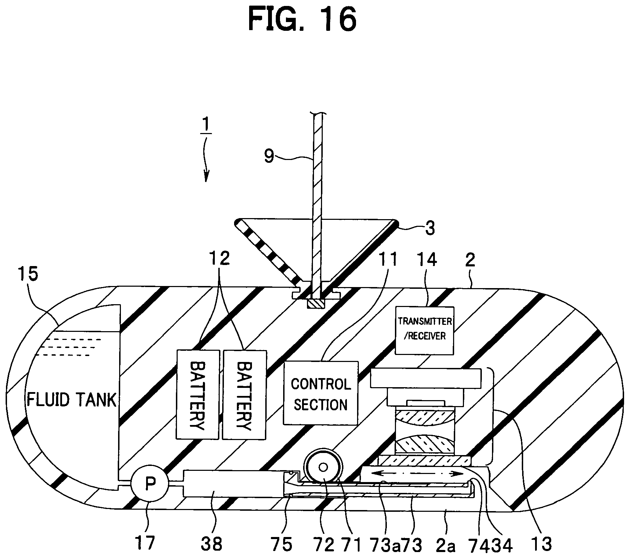

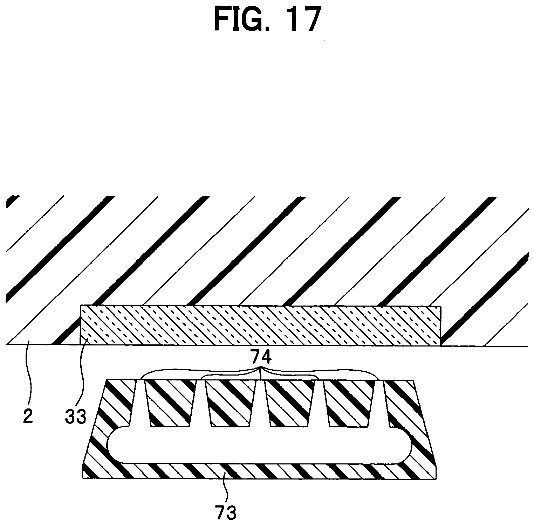

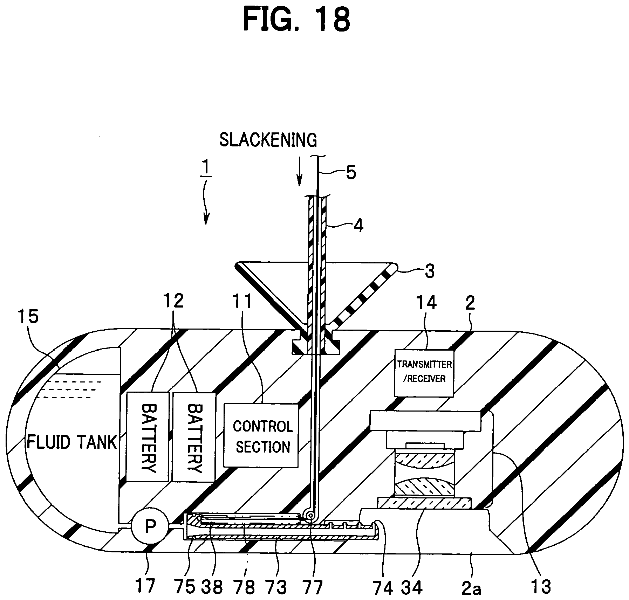

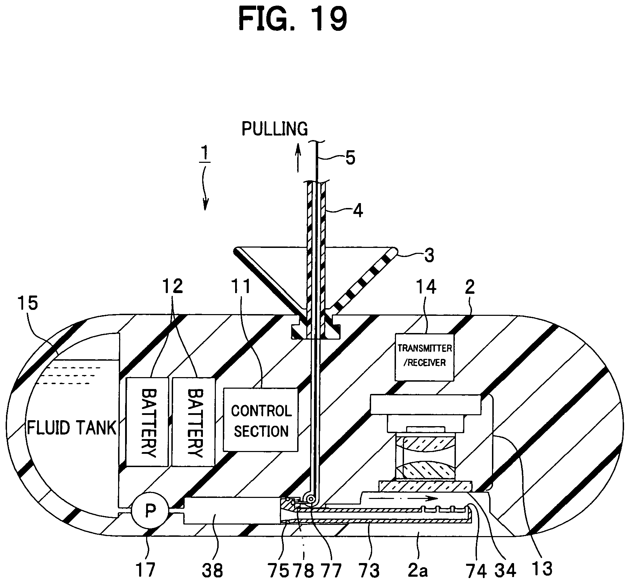

Next, a further camera set up in the abdominal cavity will be described based on Fig. 15 to Fig. 19 below. Fig. 15 is a cross-sectional view illustrating a configuration of the camera set up in the abdominal cavity, Fig. 16 is a cross-sectional view illustrating the camera set up in the abdominal cavity whose cleaning nozzle has been slidingly moved, Fig. 17 is a partial cross-sectional view illustrating a configuration of a cleaning nozzle, Fig. 18 is a cross-sectional view illustrating a configuration of a camera set up in the abdominal cavity and Fig. 19 is a cross-sectional view illustrating the camera set up in the abdominal cavity whose cleaning nozzle has been slidingly moved. The present arrangement is a modification of the arrangement according to Figs. 1 to 5 and the same components as those in the camera set up in the abdominal cavity of Figs. 1 to 5 will be assigned the same reference numerals and descriptions of those components and operations/effects will be omitted.

The camera set up in the abdominal cavity (hereinafter also simply referred to as "camera") 1 is provided with a tubular cleaning nozzle 73 slidably disposed in the camera body 2 as shown in Fig. 15 and Fig. 16. The cleaning nozzle 73 is configured to be led out into the observation window 2a and jet water, a cleaning liquid or the like over the surface of the cover lens 34 of the image pickup unit 13 for cleaning.

The cleaning nozzle 73 is made to slidingly move by being driven by a motor 71, which is drive means (drive source) incorporated in the camera body 2. To be more specific, the cleaning nozzle 73 is slidably disposed in a slide holding hole portion 38 which is left open on the side of the observation window 2a formed in the camera body 2. A gear groove 73a serving as a rack meshed with a gear 72 serving as a pinion of the motor 71 is formed at the top of the cleaning nozzle 73.

That is, as in the case of the arrangement of Figs. 1 to 5, when the micro pump 17 is driven, the motor 71 is driven/stopped by the user operating an outside controller (not shown) and the cleaning nozzle 73 slidingly moves so as to lead out into the observation window 2a. Furthermore, since the cleaning nozzle 73 is made to slidingly move by being driven by the motor 71, the camera 1 is provided with no flexible tube as in the case of the arrangement according to Figs. 11 to 13 and the wire 9 for lifting the camera body 2 extends from the suction cup 3 which is an abdominal wall fixing section.

The slide holding hole portion 38 of the camera body 2 in which the sliding cleaning nozzle 73 is accommodated communicates with the fluid tank 15 via the fluid supply pipe 16. The fluid supply pipe 16 is provided with a micro pump 17 as in the case of the arrangement of Figs. 1 to 5.

The cleaning nozzle 73 is provided with an O ring 75 contacting the inner surface of the slide holding hole portion 38 to keep the interior thereof watertight even when the cleaning nozzle 73 slidingly moves. That is, water, a cleaning liquid or the like in the fluid tank 15 sent into the slide holding hole portion 38 by being driven by the micro pump 17 flows into the cleaning nozzle 73.

The water, cleaning liquid or the like is jetted out from spouts 74 (see Fig. 17) which are openings formed at an end of the cleaning nozzle 73 on the observation window 2a side over the surface of the cover lens 34 of the image pickup unit 13. Furthermore, there are five spouts 74 here along the short side axis of the cleaning nozzle 73 so as to be able to clean the cover lens 34 evenly.

The rest of the configuration of the camera 1 is also the same as that of the camera 1 of Figs. 1 to 5.

As described so far, the camera 1 may also have a configuration in which the cleaning nozzle 73 is led out into the observation window 2a, water, a cleaning liquid or the like is jetted over the surface of the cover lens 34 of the image pickup unit 13 so as to clean fogging, deposits such as dirt.

As shown in Fig. 18 and Fig. 19, the camera 1 may also be configured such that the cleaning nozzle 73 is made to slidingly move by pulling/slackening the operation wire 5.

To be more specific, the insertion direction of the operation wire 5 is changed by a pulley 77 and the operation wire 5 is inserted into the slide holding hole portion 38 of the camera body 2. An end of the operation wire 5 is connected to the cleaning nozzle 73. A coil spring 78 is disposed in the slide holding hole portion 38 so as to externally fitted onto the operation wire 5. The coil spring 78 urges the cleaning nozzle 73 to be accommodated in the slide holding hole portion 38.

That is, when the user pulls the operation wire 5, the cleaning nozzle 73 can be led out into the observation window 2a and when the user slackens the operation wire 5, the cleaning nozzle 73 can be accommodated into the slide holding hole portion 38 by the urging force of the coil spring 78.

Here, the cleaning nozzle 73 has a configuration provided with a plurality of (here four) spouts 74 along the longitudinal direction in which the cleaning nozzle 73 is led out into the observation window 2a.

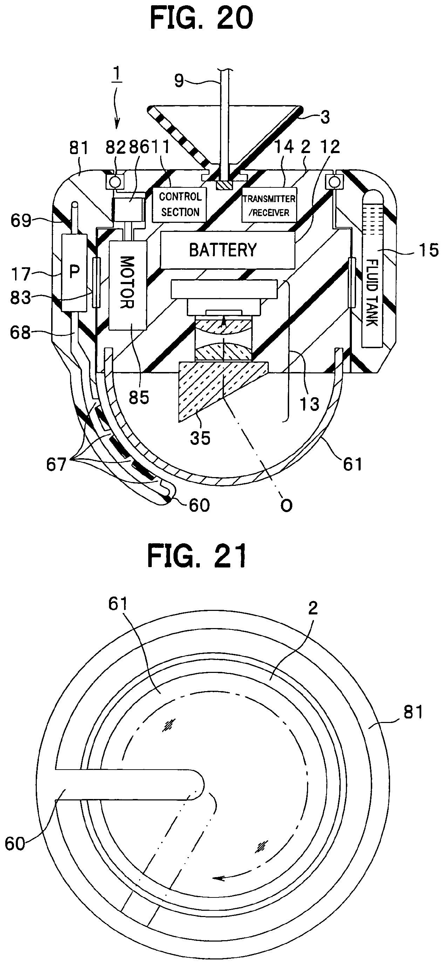

Next, a further camera set up in the abdominal cavity will be described based on Fig. 20 and Fig. 21. Fig. 20 is a cross-sectional view illustrating a configuration of the camera set up in the abdominal cavity and Fig. 21 is a plan view of the camera set up in the abdominal cavity viewed from the image taking direction. The present arrangement is a modification of the arrangement according to Figs. 11 to 13 and the same components as those in the camera set up in the abdominal cavity of Figs. 1 to 5 and Figs. 11 to 13 will be assigned the same reference numerals and descriptions of those components and operations/effects will be omitted.

As shown in Fig. 20 and Fig. 21, the camera set up in the abdominal cavity (hereinafter also simply referred to as "camera") 1 has a configuration in which the cleaning nozzle 60, which is formed integrally with the camera body 2 according to Figs. 11 to 13, rotates around the surface of the dome-shaped cover member 61 and cleans the cover member 61.

The cleaning nozzle 60 is formed integrally with a cylindrical rotation section 81 which is rotatably disposed around the camera body 2 by being held by a bearing 82. The rotation section 81 is provided with the micro pump 17 and the fluid tank 15, and the fluid supply channel 68 that extends to the cleaning nozzle 60 and the fluid supply pipe 69 in which the micro pump 17 is interposed.

Furthermore, the rotation section 81 and the camera body 2 are provided with a slip ring 83 so that a drive control signal outputted from the control section 11 to the micro pump 17 is inputted to the micro pump 17 even when the rotation section 81 rotates, and are configured so as to maintain electric connection.

The rotation section 81 is driven to rotate around the side peripheral part of the camera body 2 by a drive force of a motor 85 incorporated on the camera body 2 side. The motor 85 is provided with a roller 86 which is held in pressure contact with the inner surface of the rotation section 81. A gear may also be used instead of the roller 86, and in such a case, a gear groove meshed with the gear is formed in the inner surface of the rotation section 81.

Furthermore, the rest of the configuration of the camera 1 is the same as that of the camera 1 of Figs. 11 to 13.

As described above, the camera 1 may also have a configuration in which the cleaning nozzle 60 is rotated and water, a cleaning liquid or the like is jetted over the surface of the cover member 61 so as to clean fogging, deposits such as dirt off the surface of the cover member 61.

The fluid tank 15 may also naturally have a cassette type configuration which is attachable/detachable to/from the camera body 2 or the like.

The medical instrument can always obtain a clear observation image without interfering with observation even when removing fogging, deposits such as dirt. The present application is filed claiming the priority of Japanese Patent Application No. 2009-052367 filed in Japan on March 5, 2009,

## Claims

1. A medical instrument (1) comprising:
a camera body (2) introduced into a body;
image pickup means (13) incorporated in the camera body (2) for picking up an image of an object;
a transparent cover member (41) movably disposed at the camera body (2) that always covers an image taking side of the image pickup means (13) irrespective of a moving position;
moving means (5, 42, 43) that causes the cover member (41) to move; and
cleaning means (15, 16, 17, 18) including a cleaning nozzle (18) disposed outside an image pickup range of the image pickup means (13), the cleaning means (15, 16, 17, 18) being adapted to perform cleaning by jetting a fluid over a surface part of the cover member (41) moved to outside the image pickup range of the image pickup means (13), from the cleaning nozzle (18) opposed to the surface part,
**characterized in that** the cover member (41) is formed into a cylindrical shape, blocks an observation window (2a) formed in the camera body (2) so as to always cover the image taking side of the image pickup means (13) and rotates around the image pickup means (13).

2. The medical instrument (1) according to claim 1, wherein
a cleaning tank (19) comprising the cleaning nozzle (18) is provided outside an image pickup range of the image pickup means (13) of the camera body (2), and
the cover member (41) is adapted to move to the cleaning tank (19) and the surface part is cleaned using a fluid jetted from the cleaning nozzle (18).

3. The medical instrument according to claim 1 or 2, wherein the moving means (5, 42, 43) comprises an operation wire (5), and
the cover member (41) is made to move by pulling/slackening of the operation wire (5).

4. The medical instrument (1) according to any one of claims 1 to 3, wherein the moving means (5, 42, 43) comprises a motor, and
the cover member (41) is made to move by receiving a drive force of the motor.

5. The medical instrument (1) according to any one of claims 1 to 4, wherein a surface treatment of hydrophilic coating is applied to the surface of the cover member (41, 61).

6. The medical instrument (1) according to any one of claims 1 to 5, wherein the camera body (2) comprises a fixing section (3) for fixing to an in-body wall (107) in the body.

## Patentansprüche

1. Medizinisches Instrument (1) mit:
einem Kamerakörper (2), der in einen Körper eingeführt wird;
einer in den Kamerakörper (2) integrierten Bildaufnahmeeinrichtung (13) zur Aufnahme eines Bildes eines Objekts;
einem transparenten Abdeckelement (41), das beweglich an dem Kamerakörper (2) angebracht ist und eine Bildaufnahmeseite der Bildaufnahmeeinrichtung (13) ungeachtet einer Bewegungsposition stets abdeckt;
einer Bewegungseinrichtung (5, 42, 43), die bewirkt, dass sich das Abdeckelement (41) bewegt; und
einer Reinigungseinrichtung (15, 16, 17, 18), die eine Reinigungsdüse (18) aufweist, die außerhalb eines Bildaufnahmebereichs der Bildaufnahmeeinrichtung (13) angeordnet ist, wobei die Reinigungseinrichtung (15, 16, 17, 18) dazu ausgebildet ist, einen Reinigungsvorgang auszuführen, durch Ausstoßen eines Fluids über einem Oberflächenteil des Abdeckelements (41), der aus dem Bildaufnahmebereich der Bildaufnahmeeinrichtung (13) heraus bewegt wird, aus der dem Oberflächenteil gegenüberliegenden Reinigungsdüse (18),
**dadurch gekennzeichnet, dass** das Abdeckelement (41) zylinderförmig ausgebildet ist, ein in dem Kamerakörper (2) gebildetes Beobachtungsfenster (2a) blockiert, um die Bildaufnahmeseite der Bildaufnahmeeinrichtung (13) stets abzudecken, und sich um die Bildaufnahmeeinrichtung (13) dreht.

2. Medizinisches Instrument (1) nach Anspruch 1, wobei
ein Reinigungstank (19), der die Reinigungsdüse (18) aufweist, außerhalb eines Bildaufnahmebereichs der Bildaufnahmeeinrichtung (13) des Kamerakörpers (2) angeordnet ist, und
das Abdeckelement (41) dazu ausgebildet ist, sich zu dem Reinigungstank (19) hin zu bewegen, und der Oberflächenteil unter Verwendung eines von der Reinigungsdüse (18) ausgestoßenen Fluids gereinigt wird.

3. Medizinisches Instrument (1) nach Anspruch 1 oder 2, wobei die Bewegungseinrichtung (5, 42, 43) einen Betätigungsdraht (5) aufweist und
das Abdeckelement (41) dazu ausgebildet ist, sich durch Anziehen/Lockern des Betätigungsdrahtes (5) zu bewegen.

4. Medizinisches Instrument (1) nach einem der Ansprüche 1 bis 3, wobei die Bewegungseinrichtung (5, 42, 43) einen Motor aufweist und
das Abdeckelement (41) dazu ausgebildet ist, sich durch Aufnehmen einer Antriebskraft des Motors zu bewegen.

5. Medizinisches Instrument (1) nach einem der Ansprüche 1 bis 4, wobei eine Oberflächenbehandlung einer hydrophilen Beschichtung auf die Oberfläche des Abdeckelements (41, 61) aufgebracht wird.

6. Medizinisches Instrument (1) nach einem der Ansprüche 1 bis 5, wobei der Kamerakörper (2) einen Befestigungsbereich (3) zum Befestigen an einer körperinneren Wand (107) in dem Körper aufweist.

## Revendications

1. Instrument médical (1) comprenant :
un corps de caméra (2) introduit dans un corps ;
un moyen de prélèvement d'image (13) incorporé dans le corps de caméra (2) pour prélever une image d'un objet ;
un élément de couvercle transparent (41) disposé mobile sur le corps de caméra (2) qui couvre toujours un côté de prise d'image du moyen de prélèvement d'image (13) quelle que soit une position de mouvement ;
un moyen de déplacement (5, 42, 43) qui déplace l'élément de couvercle (41) ; et
un moyen de nettoyage (15, 16, 17, 18) comprenant une buse de nettoyage (18) disposée à l'extérieur d'une plage de prise d'image du moyen de prélèvement d'image (13), le moyen de nettoyage (15, 16, 17, 18) étant adapté pour effectuer le nettoyage en injectant un fluide sur une partie de surface de l'élément de couvercle (41) déplacé vers l'extérieur de la plage de prise d'image du moyen de prélèvement d'image (13), à partir de la buse de nettoyage (18) opposée à la partie de surface,
**caractérisé en ce que** l'élément de couvercle (41) a une forme cylindrique, bloque une fenêtre d'observation (2a) formée dans le corps de caméra (2) de façon à toujours couvrir le côté de prise d'image du moyen de prélèvement d'image (13) et tourne autour du moyen de prélèvement d'image (13).

2. Instrument médical (1) selon la revendication 1, dans lequel
un réservoir de nettoyage (19) comprenant la buse de nettoyage (18) est prévu à l'extérieur d'une plage de prise d'image du moyen de prélèvement d'image (13) du corps de caméra (2), et
l'élément de couvercle (41) est adapté pour se déplacer vers le réservoir de nettoyage (19) et la partie de surface est nettoyée en utilisant un fluide injecté à partir de la buse de nettoyage (18).

3. Instrument médical (1) selon la revendication 1 ou 2, dans lequel le moyen de déplacement (5, 42, 43) comprend un câble de fonctionnement (5), et
l'élément de couvercle (41) se déplace par la traction/le relâchement du câble de fonctionnement (5).

4. Instrument médical (1) selon l'une quelconque des revendications 1 à 3, dans lequel le moyen de déplacement (5, 42, 43) comprend un moteur, et
l'élément de couvercle (41) se déplace en recevant une force d'entraînement du moteur.

5. Instrument médical (1) selon l'une quelconque des revendications 1 à 4, dans lequel un traitement de surface de revêtement hydrophile est appliqué sur la surface de l'élément de couvercle (41, 61).

6. Instrument médical (1) selon l'une quelconque des revendications 1 à 5, dans lequel le corps de caméra (2) comprend une section de fixation (3) pour la fixation à une paroi enchâssée (107) dans le corps.
